# EUROPÄISCHE PATENTSCHRIFT

(11) **EP 0 493 658 B1**
(45) Veröffentlichungstag und Bekanntmachung des Hinweises auf die Patenterteilung: **01.09.1993**
(21) Anmeldenummer: 91118027.1
(22) Anmeldetag: 23.10.1991
(51) Int. Cl.: C02F 1/50, C07C 45/51, C07C 47/22

(54) **Verfahren zur Dotierung wässriger Lösungen mit Acrolein in biozid-wirksamer Konzentration**
Method of doping aqueous solutions with acrolein at a sufficient concentration level to be active as a biocide
Procédé de dopage de solutions aqueuses avec de l'acroléine à une concentration suffisante pour être active comme biocide

(30) Priorität: 03.12.1990 DE 4038471
(43) Veröffentlichungstag der Anmeldung: 08.07.1992
(73) Patentinhaber: DEGUSSA AG, 60311 Frankfurt (DE)
(72) Erfinder: Werle, Peter, Dr., W-6460 Gelnhausen 2 (DE); Trageser, Martin, W-6460 Gelnhausen-Hoechst (DE); Piana, Hermann, Dr., W-8755 Alzenau (DE)

(56) Entgegenhaltungen:
- EP-A- 0 022 697
- US-A- 4 851 583

## Beschreibung

Die Erfindung richtet sich auf ein Verfahren zur Dotierung wäßriger Lösungen, insbesondere Wasserkreisläufen, mit Acrolein in biozid wirksamer Konzentration, wobei Acrolein durch Deacetalisierung von offenkettigen oder cyclischen Acroleinacetalen in Gegenwart stark saurer Katalysatoren in wäßriger Phase gebildet, aus der genannten wäßrigen Phase abgetrieben und in die zu dotierende wäßrige Lösung überführt wird.

Acrolein ist ein wohlbekanntes Biozid, um Flüssigkeiten, insbesondere wäßrige Lösungen offener und geschlossener Kreilaufsysteme, welche schleimbildende Mikroorganismen enthalten, zu behandeln - siehe beispielsweise R. Howell et al., Paper Trade Journal 160 (1976), Seiten 40-43. Die biozide Wirksamkeit von Acrolein richtet sich auf die Verhütung, Regulierung und Zerstörung von Mikroorganismen aus der Reihe der Bakterien, Viren, Pilze und Algen; die hohe Effektivität von Acrolein als Pestizid gestattet es, Acrolein in sehr niedriger Anwendungskonzentration in Wasser zu verwenden - US-PS 2,959,476 und US-PS 3,250,667.

Trotz hoher biozider Wirksamkeit von Acrolein stehen einer breiten Verwendung seine Gefahreneigenschaften entgegen. Aufgrund seiner hohen Reaktionsfähigkeit, seiner Neigung bei unsachgemäßer Handhabung spontan und explosionsartig zu polymerisieren, seiner starken Reizwirkung auf die Atmungsorgane und die Augen sowie seiner trotz Stabilisierung begrenzten Lagerfähigkeit sind besondere Sicherheitsmaßnahmen bei der Handhabung unerläßlich und erfordern ein geschultes Personal. Es hat daher nicht an Versuchen gefehlt, Acrolein in Form einer leichter und sicher handhabbaren, lagerbeständigen sowie weniger toxischen Depotverbindung, eines sogenannten Acroleinabspalters, einzusetzen.

Acroleinacetale können als Depotverbindung angesehen werden, aus welchen durch säurekatalysierte Deacetalisierung Acrolein freigesetzt wird - US-PS 4,851,583. Acroleinacetale niederer Alkohole, wie Acroleindimethyl- und -diethylacetal sind zwar selbst noch biozid wirksam (US-PS 3,298,908, US-PS 3,690,857), jedoch ist ihre Handhabung immer noch mit Risiken behaftet. Demgegenüber sind Acroleinacetale längerkettiger oder mehrwertiger Alkohole in den üblicherweise vorliegenden schwach sauren, neutralen oder schwach basischen Medien per se als Biozid praktisch wirkungslos, weil eine Acroleinabspaltung hier nicht oder nur in untergeordnetem Maße erfolgt; andererseits sind solche Acetale nahezu geruchlos, relativ untoxisch und problemlos zu handhaben.

Die französische Patentschrift 1 546 472 beschreibt ein Verfahren zur Reinigung von Glycerin, das cyclische Acroleinglycerinacetale enthält. Hierbei erfolgt eine Behandlung mit sauren Ionenaustauschern, gefolgt von einer solchen mit Hydrogensulfitgruppen dotierten Anionenaustauschern. Die Deacetalisierung zum Zwecke der Gewinnung von Acrolein und Dotierung wäßriger Lösung wird nicht offenbart.

Gemäß dem Verfahren der US-Patentschrift 4,851,583 werden Acroleinacetale der allgemeinen Formel 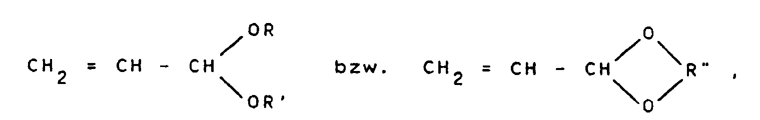
worin R und R' eine C₁- bis C₆-Alkylgruppe und R'' der Rest eines zwei- oder mehrwertigen Alkohols, etwa eines 1,2-Glykols oder Glycerin, bedeutet, unter Verwendung stark saurer Ionenaustauscher gespalten; die Verwendung von Mineralsäuren wurde als unbefriedigend dargestellt. Die bei der Deacetalisierung gewonnenen, Acrolein und den Alkohol enthaltenden und damit pestizid wirksamen Lösungen werden zur Behandlung von Wasser eingesetzt. Die Anmelderin der vorliegenden Erfindung hat das Beispiel 2 der US-PS 4,851,583 unter Verwendung des nichttoxischen Acroleinglycerinacetals (ein Gemisch aus cis- und trans- 2-Vinyl-4-hydroxymethyl-1,3-dioxolan und cis- und trans-2-Vinyl-5-hydroxy-1,3-dioxan) nachgearbeitet und gefunden, daß in dem angegebenen Zeitraum nur etwa 40 % des Acetals gespalten worden sind. Nachteile des vorbekannten Verfahrens sind somit:
- die unvollständige Ausnutzung des eingesetzten Acetals, wodurch die Wirtschaftlichkeit desselben für den Einsatz als Biozid erheblich gemindert wird;
- die eingesetzte niedrige Konzentration an Acroleinacetal (0,1 - 0,16 Gew.-%);
- die sehr große Menge an Ionenaustauscher bezogen auf eingesetztes Acroleinacetal;
- die Verwendung von sehr teurem perfluoriertem Ionenaustauscher mit Sulfonatgruppen;
- Überführung auch des Alkohols des Acroleinacetals in das biozid zu behandelnde Wasser und damit Erhöhung des Gehalts an organischer Belastung in diesem.

Aufgabe der vorliegenden Erfindung ist demgemäß, ein Verfahren zur Dotierung von wäßrigen Lösungen mit Acrolein in biozid wirksamer Konzentration aufzuzeigen, das die Nachteile des vorbekannten Verfahrens nicht aufweist.

Gefunden wurde ein Verfahren zum Dotieren wäßriger Lösungen mit Acrolein in biozid wirksamer Konzentration, wobei das Acrolein durch Deacetalisierung von Acroleinacetalen in wäßriger Phase in Gegenwart eines stark sauren Katalysators gebildet worden ist, das dadurch gekennzeichnet ist, daß man das während der Deacetalisierung gebildete Acrolein ständig aus der genannten wäßrigen Phase entfernt und in die zu dotierende wäßrige Lösung überführt, indem man einen durch die genannte wäßrige Phase geleiteten und dabei mit Acrolein dotierten inerten Gasstrom in die zu dotierende wäßrige Lösung einleitet, welche das Acrolein aus dem Gasstrom herauslöst, oder indem man den Druck über der genannten wäßrigen Phase unter Verwendung einer Flüssigkeitsstrahlpumpe, deren Treibmittel die zu dotierende wäßrige Lösung oder dieser zuzusetzendes Wasser ist, worin sich das abgezogene Acrolein löst, erniedrigt und, soweit erforderlich, das mit Acrolein dotierte Treibmittel der zu dotierenden wäßrigen Lösung zumischt.

Die Unteransprüche richten sich auf bevorzugte Ausführungsformen.

Bei den erfindungsgemäß einzusetzenden Acroleinacetalen handelt es sich um offenkettige und cyclische Acetale, wie sie aus der US-Patentschrift 4,851,583 bekannt sind. Bevorzugt werden solche Acroleinacetale eingesetzt, deren Alkoholkomponente ausreichend weit, also mindestens 20 °C, vorzugsweise über 40 °C, oberhalb des Siedepunkts von Acrolein siedet. Unter den einwertigen Alkoholen werden primäre Alkohole mit 3 bis 5 C-Atomen bevorzugt. Alkohole mit 2 und mehr Hydroxylgruppen, insbesondere 2 und 3 OH-Gruppen, enthalten vorzugsweise 2 bis 6 C-Atome. 1,2- und 1,3-Diole mit 2 bis 4 C-Atomen, Triole vom Typ Glycerin und Trimethylolethan oder -propan sowie Pentaerythrit werden als Alkoholkomponente für cyclische Acroleinacetale mit einer 1,3-Dioxolan- oder 1,3-Dioxan-Ringstruktur bevorzugt. Verwendbar sind auch Gemische von Acroleinacetalen, etwa solche, welche aus der Acetalisierung von Acrolein mit Glycerin zugänglich sind. Die Herstellung der Acetale ist an sich bekannt - beispielhaft wird auf die US-PS 3,014,924 verwiesen. Zweckmäßigerweise werden Acroleinacetale mit möglichst niedriger Toxizität als Quelle für Acrolein herangezogen - hierzu gehören z. B. die cyclischen Acroleinglycerinacetale.

Die Deacetalisierung läßt sich in wäßriger Phase bei Temperaturen im Bereich von 0 bis 100 °C, vorzugsweise aber bei 10 bis 50 °C und insbesondere bei 20 bis 40 °C durchführen. Die Bildung von Acrolein wird bei höherer Temperatur zwar beschleunigt, gleichzeitig können aber unerwünschte Nebenreaktionen eintreten, welche zu einem Verlust an für die Dotierung verfügbarem Acrolein führen.

Die Deacetalisierung wird durch stark saure Katalysatoren katalysiert. Die Säure kann in fester Form oder in gelöster Form zur Anwendung gelangen. Unter den gelösten Säuren sind Mineralsäuren, wie insbesondere Schwefelsäure und Phosphorsäure, ferner starke organische Säuren, wie Sulfonsäuren und perfluorierte Carbonsäuren gut geeignet. Die genannten starken Säuren können auch an festen Trägern, wie z. B. Kieselsäuren und Silikaten gebunden sein. Bei den Säuren in fester Form handelt es sich bevorzugt um stark saure organische und anorganische Ionenaustauscher, insbesondere sulfonatgruppenhaltige Austauscherharze auf der Basis einer Styrol/Divinylbenzol Polymermatrix oder eines polymeren Organosiloxans (siehe DE-PS en 32 26 093 und 35 18 881). Es ist klar, daß die Geschwindigkeit der Arcoleinfreisetzung bei der Deacetalisierung durch Erniedrigung des Mengenverhältnisses Acroleinacetal zu Katalysator erhöht werden kann.

Während es nach dem vorbekannten Verfahren zur Deacetalisierung der Verwendung eines sehr teuren Ionenaustauschers in großer Menge bedurfte - 1 g Nafionharz pro 67 mg Acroleindiethylacetal in 40 ml Wasser gemäß Beispiel 2 der US-PS 4,851,583 - konnten erfindungsgemäß beliebige starke Säuren in geringerer Menge eingesetzt werden. Entscheidend ist, daß das gebildete Acrolein möglichst unmittelbar nach seiner Bildung aus der wäßrigen Phase des Deacetylierungsgemischs entfernt wird; auf diese Weise gelingt es, Acrolein nahezu quantitativ aus dem Acetal freizusetzen und in die zu dotierende wäßrige Lösung zu überführen. Im Reaktor der Deacetalisierung verbleibt nach vollständiger Deacetalisierung und Entfernung des Acroleins eine wäßrige Lösung der Alkoholkomponenten des Acetals. Lediglich im Falle von Alkoholen mit niedrigem Siedepunkt, etwa Ethanol und Propanol, können Anteile des Alkohols mit in die zu dotierende Lösung überführt werden.

Die Überführung des freigesetzten Acroleins in die damit zu dotierende wäßrige Lösung läßt sich problemlos dadurch bewerkstelligen, daß ein inerter Gasstrom, insbesondere Stickstoff oder Luft, durch die wäßrige Phase des Deacetalisierungsgemischs geleitet wird; der Gasstrom nimmt dabei das Acrolein auf und gibt es beim Einleiten in die zu dotierende wäßrige Lösung an diese ab. Das Durchleiten des inerten Gasstroms, worunter auch ein Durchsaugen verstanden wird, kann bei Normaldruck oder vermindertem Druck erfolgen.

Gemäß einer alternativen Ausführungsform wird das Acrolein unter vermindertem Druck aus dem Deacetalisierungsreaktor unter Verwendung einer Flüssigkeitsstrahlpumpe in die zu dotierende Lösung überführt. Die Flüssigkeitsstrahlpumpe üblicher Bauart, wie beispielsweise sogenannten Wasserstrahlpumpen, wird vorzugsweise mit der zu dotierenden wäßrigen Lösung als Treibstrahl betrieben; das Acrolein löst sich somit sofort in Treibstrahl auf. Der mit Acrolein dotierte Treibstrahl wird, falls dies erforderlich ist, mit undotierter oder zu gering dotierter Lösung vermischt, um den gewünschten Dotierungsgrad einzustellen bzw. aufrechtzuerhalten.

Das erfindungsgemäße Verfahren kann zur Behandlung offener oder geschlossener Wassersysteme Anwendung finden. Kreislaufsysteme mit großen Wassermengen finden sich u. a. bei Kraftwerken, Raffinerien, in der Papierindustrie, Kaolinindustrie, bei der Erdöl- oder Erdgasexploration. Die geeignete, biozid wirksame Konzentration an Acrolein liegt üblicherweise im Bereich von 5 bis 10 ppm. Im allgemeinen muß Acrolein so dosiert werden, daß einerseits eine ausreichende biozide Depotwirkung über einen längeren Zeitraum erhalten bleibt, andererseits aber keine durch Acrolein verursachten Probleme auftreten.

Der besondere und nicht vorhersehbare Vorteil des erfindungsgemäßen Verfahrens liegt darin, daß es mit geringem technischen Aufwand möglich ist, die Dotierung bedarfsgerecht und sicher vorzunehmen und dabei das eingesetzte Acetal praktisch quantitativ auszunutzen. Die Reaktorgröße für die Deacetalisierung wird man an die zu dotierende Wassermenge und den gewünschten Dotierungsgrad anpassen; das Acroleinacetal kann in beliebiger Konzentration bis zu 50 Gew.-% in Wasser zum Einsatz kommen, so daß im allgemeinen nur kleine Reaktoren benötigt werden. Wie bereits ausgeführt, kann auch bei vorgegebener Menge Acetal die Menge gebildetes Acrolein pro Zeiteinheit über das Mengenverhältnis Acetal zu Katalysator einfach gesteuert werden.

### Vergleichsbeispiel

Gemäß Beispiel 2 der US-PS 4,851,583 wurden 100 ml einer 0,17 gew.-%igen Lösung von Acroleinglycerinacetalen in VE-Wasser (pH 6,0) mit 2,5 g Nafion 417® versetzt und bei 20 °C 15 Minuten gerührt.

Die Lösung wurde anschließend gaschromatographisch untersucht. Es wurden 0,029 g Acrolein und 0,101 g Acroleinglycerinacetale in der Lösung nachgewiesen, dies sind 39,6 % der Theorie Ausbeute an Acrolein und noch 59,4 % der eingesetzten Menge an Acroleinglycerinacetalen. Die Genauigkeit der Meßmethoden beträgt ± 3 %.

### Beispiel 1

500 g einer 10 gew.-%igen wäßrigen Acroleinglycerinacetallösung werden mit 10 g feuchtem Ionenaustauscher vom Typ Lewatit® SC 104 (H⁺-Form) (Fa. Bayer AG) versetzt. Das Reaktionsgefäß wird auf 40 °C erwärmt und ein Wasserstrahlpumpenvakuum angelegt. Dabei destilliert ein Großteil des gebildeten Acroleins ab, der Druck stellt sich nach 15 Minuten auf etwa 100 mbar ein. Im Verlauf von 30 Minuten erniedrigt sich der Druck weiter auf ca. 60 bis 70 mbar. Das abdestillierte Acrolein-Wassergemisch wird in einer mit flüssigem Stickstoff gekühlten Falle aufgefangen und nach Verdünnen mit Wasser gaschromatographisch bestimmt. Es wurden 20,0 g Acrolein gefunden; das entspricht ca. 93 % d. Th. Der Kolben selbst enthielt Glycerin neben Spuren von Acroleinglycerinacetal.

### Beispiel 2

400 g einer 20 gew.-%igen, wäßrigen Acroleinglycerinacetallösung werden mit 3 ml 96 gew.-%iger Schwefelsäure versetzt. Durch die auf 50 °C erwärmte Lösung wird ein Stickstoffstrom von ca. 0,5 l/min. geleitet und das sich bildende Acrolein ausgetrieben. Die Reaktion wird nach 1,5 Stunden abgebrochen. In der tiefgekühlten Vorlage wurde gaschromatographisch ein Acroleinanteil von 32,5 g gefunden. Das sind 94 % der Theorie. Im Deacetalisierungskolben ließ sich gaschromatographisch Acroleinglycerinacetal noch zu etwa 0,5 % nachweisen.

### Beispiel 3

### Dotierung eines 20 m³ Kühlwasserkreislaufs

In einem 2 l Reaktionsgefäß wurden 1160 g einer 20 gew.-%igen wäßrigen Acroleinglycerinacetallösung bei 20 °C vorgelegt, mit 45 g feuchtem Ionenaustauscher vom Typ Lewatit® SC 104 (H⁺-Form) versetzt und das sich bildende Acrolein im Verlauf von 1,5 Stunden bei 40 °C Innentemperatur - unter gleichzeitiger Erniedrigung des Druckes mittels einer Wasserstrahlpumpe - auf ca. 60 bis 70 mbar gasförmig in die Wasserstrahlpumpe abgezogen.

Die Wasserstrahlpumpe wurde dabei mit einem Wasserstrom des zu dotierenden 20 m³-Kreislaufs mit einem Wert von 6,8 druckseitig mit 5 bar betrieben und der Auslauf der Pumpe, in dem sich die abgesaugten gasförmigen Acrolein- bzw. Acrolein-Wassergemische gelöst haben, dem Kreislauf zurückgeführt.

Nach Beendigung der Dotierung wurde im Kühlwasserkreislauf mittels photometrischer Analysenmethode (Farbreaktion mit Dinitriophenylhydrazin) ein Gehalt von 4,5 ppm Acrolein gemessen.

Im Sumpf des Reaktionsgefäßes wurden gaschromatographisch neben Glycerin als Hauptkomponente noch 0,4 Gew.-% Acroleinglycerinacetal gefunden.

## Patentansprüche

1. Verfahren zum Dotieren wäßriger Lösungen mit Acrolein in biozid wirksamer Konzentration, wobei das Acrolein durch Deacetalisierung von Acroleinacetalen in wäßriger Phase in Gegenwart eines stark sauren Katalysators gebildet worden ist,
dadurch gekennzeichnet,
daß man das während der Deacetalisierung gebildete Acrolein ständig aus der genannten wäßrigen Phase entfernt und in die zu dotierende wäßrige Lösung überführt,
indem man einen durch die genannte wäßrige Phase geleiteten und dabei mit Acrolein dotierten inerten Gasstrom in die zu dotierende wäßrige Lösung einleitet, welche das Acrolein aus dem Gasstrom herauslöst,
oder indem man den Druck über der genannten wäßrigen Phase unter Verwendung einer Flüssigkeitsstrahlpumpe, deren Treibmittel die zu dotierende wäßrige Lösung oder dieser zuzusetzendes Wasser ist, worin sich das abgezogene Acrolein löst, erniedrigt und, soweit erforderlich, das mit Acrolein dotierte Treibmittel der zu dotierenden wäßrigen Lösung zumischt.

2. Verfahren nach Anspruch 1,
dadurch gekennzeichnet,
daß man als Quelle für Acrolein ein Acroleindi-n-alkylacetal, deren Alkylgruppen 3 bis 5 C-Atome enthalten, oder ein cyclisches Acroleinacetal, deren Alkoholkomponente 2 bis 6 C-Atome und 2 bis 6, vorzugsweise 2 bis 4, OH-Gruppen aufweist, einsetzt.

3. Verfahren nach Anspruch 1 oder 2,
dadurch gekennzeichnet,
daß man als Deacetalisierungskatalysator stark saure anorganische oder organische Ionenaustauscher verwendet, insbesondere sulfonatgruppenhaltige Austauscherharze auf der Basis einer Styrol/Dinvynlbenzol-Polymermatrix oder polymerer Organosiloxane.

4. Verfahren nach Anspruch 1 oder 2,
dadurch gekennzeichnet,
daß man als Deacetalisierungskatalysator Mineralsäuren, vorzugsweise Schwefelsäure, einsetzt.

## Claims

1. A process for doping aqueous solutions with acrolein in biocidally active concentrations, the acrolein having been formed by the deacetalisation of acrolein acetals in the aqueous phase in the presence of a strongly acid catalyst, characterised in that
the acrolein formed during deacetalisation is continuously removed from the aforesaid aqueous phase and transferred to the aqueous solution which is to be doped
by introducing an inert stream of gas which has been passed through the said aqueous phase and is thus doped with acrolein into the aqueous solution to be doped, which extracts the acrolein from the gas stream,
or by lowering the pressure over the aforesaid aqueous phase by means of a liquid jet pump whose propellant consists of the aqueous solution to be doped or of the water to be added thereto, in which the acrolein which has been removed dissolves, and adding, as far as necessary, the propellant doped with acrolein to the aqueous solution to be doped.

2. A process according to Claim 1, characterised in that the source of acrolein used is an acrolein-di-n-alkylacetal whose alkyl groups have 3 to 5 carbon atoms or a cyclic acrolein acetal whose alcohol component has from 2 to 6 carbon atoms and from 2 to 6, preferably from 2 to 4, OH groups.

3. A process according to Claim 1 or Claim 2, characterised in that the deacetalisation catalyst used consists of strongly acid inorganic or organic ion exchangers, in particular sulphonate group-containing exchanger resins based on a styrene/divinylbenzene polymer matrix or polymeric organosiloxanes.

4. A process according to Claim 1 or Claim 2, characterised in that the deacetalisation catalyst used consists of mineral acids, preferably sulphuric acid.

## Revendications

1. Procédé de dopage de solutions aqueuses par l'acroléine en concentration efficace pour être biocide, dans lequel l'acroléine est produite par désacétalisation d'acétals d'acroléine en phase aqueuse en présence d'un catalyseur fortement acide, caractérisé
- en ce qu'on élimine en continu de la phase aqueuse citée l'acroléine formée pendant la désacétalisation et qu'on la transfère dans la solution aqueuse à doper,
- en introduisant dans la solution aqueuse à doper un courant de gaz inerte qui a passé dans la phase aqueuse citée et s'est ainsi chargé d'acroléine, la solution à doper dissolvant l'acroléine contenue dans le courant gazeux, ou
- en diminuant la pression au-dessus de la phase aqueuse citée en utilisant une pompe à jet de liquide, dont l'agent moteur est constitué par la solution aqueuse à doper ou l'eau qu'on doit lui ajouter, dans laquelle l'acroléine extraite se dissout, décroît et, si c'est nécessaire, on mélange le courant de jet dopé à l'acroléine à la solution aqueuse à doper.

2. Procédé selon la revendication 1, caractérisé en ce que comme source d'acroléine, on utilise un acroléine- n-alkylacétal, dont les groupes alkyles comprennent 3 à 5 atomes de C, ou un acétal d'acroléine cyclique, dont la composante alcool présente 2 à 6 atomes de C et 2 à 6, de préférence 2 à 4 groupes OH.

3. Procédé selon la revendication 1 ou 2, caractérisé en ce qu'on utilise comme catalyseur de désacétalisation un échangeur ionique organique ou minéral fortement acide, notamment des résines échangeuses contenant des groupes sulfonates à base d'une matière polymère styrène/divinylbenzène ou des organosiloxanes polymères.

4. Procédé selon la revendication 1 ou 2, caractérisé en ce qu'on utilise comme catalyseur de désacétalisation des acides minéraux, de préférence de l'acide sulfurique.
